# EUROPÄISCHE PATENTSCHRIFT

(11) **EP 0 591 966 B1**
(45) Veröffentlichungstag und Bekanntmachung des Hinweises auf die Patenterteilung: **31.07.2002**
(21) Anmeldenummer: 93116239.0
(22) Anmeldetag: 07.10.1993
(51) Int. Cl.: A61B 8/12

(54) **Endoluminales Ultraschallgerät und seine Anwendung**
Endoluminal ultrasonic device and its application
Appareil endoluminal à ultrasons et son application

(30) Priorität: 09.10.1992 US 959150
(43) Veröffentlichungstag der Anmeldung: 13.04.1994
(73) Patentinhaber: ACOUSTIC IMAGING TECHNOLOGIES CORPORATION, Phoenix, Arizona 85044 (US)
(72) Erfinder: Slayton, Michael H., Jr., Tempe, Arizona 85284 (US)
(74) Vertreter: Hummel, Adam

(56) Entgegenhaltungen:
- EP-A- 0 429 799
- WO-A-91/19458
- WO-A-92/03095
- WO-A-92/11055

## Beschreibung

Die Erfindung bezieht sich auf ein endoluminales Ultraschallgerät gemäß dem Oberbegriff des Patentanspruchs 1.

Ein Typ dieses Geräts weist einen einzelnen rotierenden Ultraschallwandler oder einen Ultraschallreflektor am Ende des flexiblen stationären Katheters auf. Das Wandlerelement oder der Reflektor rotiert um den Katheter und führt somit eine Abtastung senkrecht zur Katheterachse aus.

Bei einem anderen Gerätetyp eines endoluminalen Ultraschallgeräts sind mehrere stationäre Ultraschall-Wandlerelemente um die Peripherie am Ende eines flexiblen stationären Katheters angeordnet. Die Wandlerelemente werden sequentiell in Gruppen aktiviert, um eine Abtastung in einer einfachen vertikalen Ebene zur Katheterachse durchzuführen. Dieser Gerätetyp wurde in der US-PS 3,938,505 beschrieben. Da er sich nur auf eine Ebene senkrecht zur Katheterachse beschränkt, wetsen beide beschriebenen Geräte keine gute Längs-, d.h. Axial-Auflösung auf.

Das US-Patent 5,070,879 beschreibt ein manuell gehaltenes transrektales Ultraschallgerät, das in einem starren Längsgehäuse integriert ist. In dem Gehäuse wird ein Ultraschall-Wandler-Elementen-Array axial montiert für die Schwingungen um die Längsachse.

Aus der WO-A-92/11055 ist ein Katheter zur Ultraschall-Bildaufnahme bekannt, welches eine flexible Katheterhülle aufweist, die in ihrem Inneren außer einem Führungsdraht einen flexiblen Antriebsschaft aufnimmt, an dessen distalem Ende sich eine Ultraschall-Wandler-Sonde befindet. An seinem proximalen Ende ist dieser Antriebsschaft über eine Spindel mit einem Antriebsmotor verbun-den, mit dessen Hilfe der Antriebsschaft sowie die Ultraschall-Wandler-Sonde in Drehbewegungen versetzt werden kann. Die Katheterhülle selbst bleibt währenddessen in Ruhe.

Demgegenüber besteht ein wesentlicher Punkt des erfindungsgemäßen endoluminalen Ultraschallgerätes darin, daß es mit einem Antrieb mit drehbarer Antriebswelle ausgestattet ist, die hin und her schwingt, wobei eine flexible Katheterhülle an ein Antriebsende gekoppelt ist und mit der Antriebswelle schwingt. Die Katheterhülle hat eine Achse, um die die Antriebswelle schwingt. Der Durchmesser ist dergestalt, daß sie in ein menschliches Blutgefäß eingeführt werden kann. Ein Ultraschallwandler wird an das andere Ende der Katheterhülle integriert und schwingt mit diesem. Der Wandler ist mit einem Array an autonomen achsparallel angeordneten Elementen ausgestattet und bildet somit einen senkrecht zur Achse rotierenden Strahl. Die Wandlerelemente werden durch eine Elektronik in der Nähe des Antriebs angesteuert. Anhand von durch die Katheterhülle durchgeführten Leitungen werden die Wandlerelemente an die Elektronik gekoppelt. In dieser bevorzugten Ausführungsart steuert die Elektronik die Wandler sukzessiv an, um Bildabtastungen in gewünschten unterschiedlichen Winkeln um die Achse herum auszuführen, wobei die elektrische Verkabelung aus mehreren Leitungen besteht, die einzeln die Wandlerelemente an die Elektronik koppeln. In der Elektronik ist eine elektronische Strahlfokussierung zur Verbesserung der Axialauflösung des Geräts integriert.

Die Erfindung ermoglicht ein Verfahren zur Ultraschall-Bilddarstellung von Körperhohlräumen. Ein Array mit entlang der Achse angeordneten Ultraschall-Wandlerelementen wird in ein BlutgefäB oder in einen anderen Körperhohlraum mit Flüssigkeit eingeführt, der abgebildet werden soll. Die vom Array ausgehenden Leitungen werden zur Steuerung der Arrayfunktion durch die Katheterhülle an die elektronischen Schaltkreise außerhalb des menschlichen Körpers geführt.

Die Katheterhülle und das Wandlerarray werden zusammen im Blutgefäß oder in einem anderen Körperhohlraum während des Betriebs des Arrays in Schwingung versetzt, um Ultraschallimpulse durch die Flüssigkeit an die umgebende Gewebewand abzugeben. Die Echosignale werden verarbeitet und am Display ausgegeben, so daß die den Körperhohlraum umgebende Gewebestruktur untersucht werden kann.

Die Merkmale der speziellen Ausführungsarten zur Ermittlung des optimalen Verfahrens zur Realisierung der Erfindung sind in den Zeichnungen dargestellt. Es zeigen:
- Fig. 1: eine Ansicht eines medizinischen Ultraschallgeräts mit endoluminarem Gerät, in dem die Prinzipien der Erfindung integriert sind,
- Fig. 2: den Schnitt des Katheterendes von Fig. 1 mit dem Ultraschallwandler und den elektrischen Anschlüssen,
- Fig. 3: die Schnittansicht des Ultraschallwandlers auf Ebene A-A in Fig. 2,
- Fig. 4: die perspektivische Ansicht des Katheterendes und des Ultraschallwandlers und
- Fig. 5: ein schematisches Blockdiagramm des gesamten Ultraschall-Bilddarstellung-Systems mit integriertem Ultraschallwandler und Katheter.

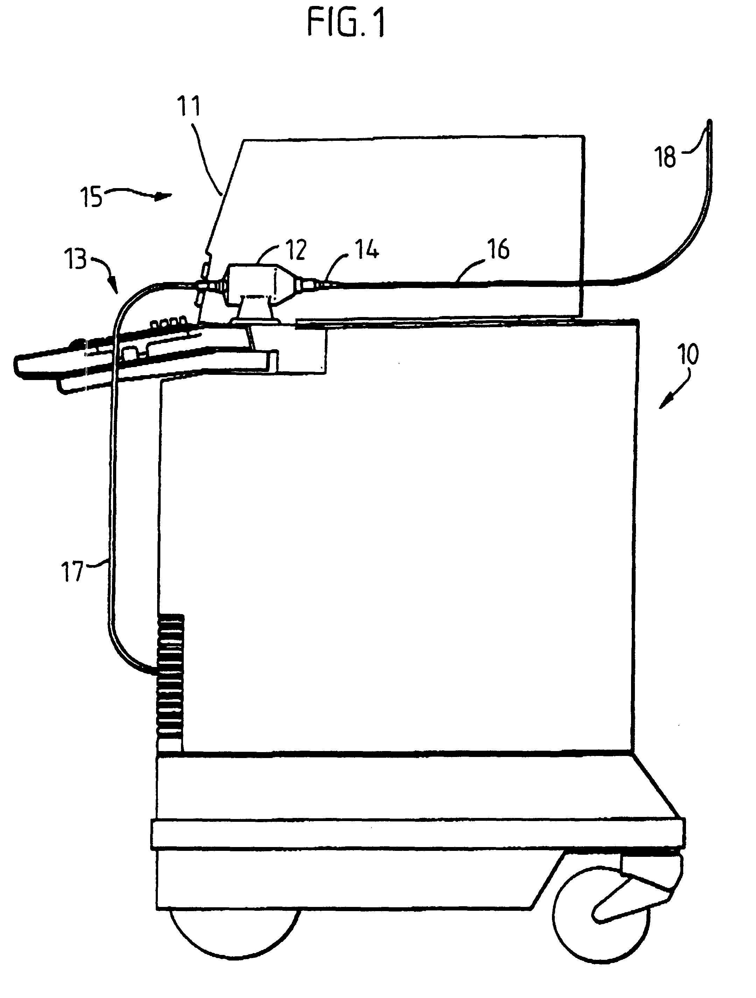

In Fig. 1 wird ein medizinisches Ultraschall-Bildsystem mit Konsole 10 dargestellt. Wie aus Fig. 1 ersichtlich, ist das Bedienpult 11 auf dem schrägen Kontrollpanel auf der linken Seite der Konsole 10 angeordnet. Ein Antrieb 12 wird neben dem Bedienpult 11 auf der Konsole 10 montiert. Dieser Antrieb 12 hat eine hohle rotierende Antriebswelle 14, die sich vorzugsweise in einem Winkel von 360° dreht. Ein Video-Ausgabeterminal 15 ist vor dem Bedienpult 11 angeordnet. Das proximale Ende einer flexiblen Katheterhülle 16 wird an die Antriebswelle 14 angeschlossen und somit in Bewegung gesetzt. Der Durchmesser der Katheterhülle ist so gestaltet, daß er in ein menschliches Blutgefäß, z.B. mit einer Größe von 2,25 mm oder in einen anderen Körperhohlraum mit Flüssigkeit, wie z.B. die Harnröhre eingeführt werden kann. Eine Ultraschall-Wandler-Sonde wird an das distale Ende der Katheterhülle 16 angeschlossen. Ein Signalkabel 17 wird vom Antrieb 12 in die Konsole 10 geführt.

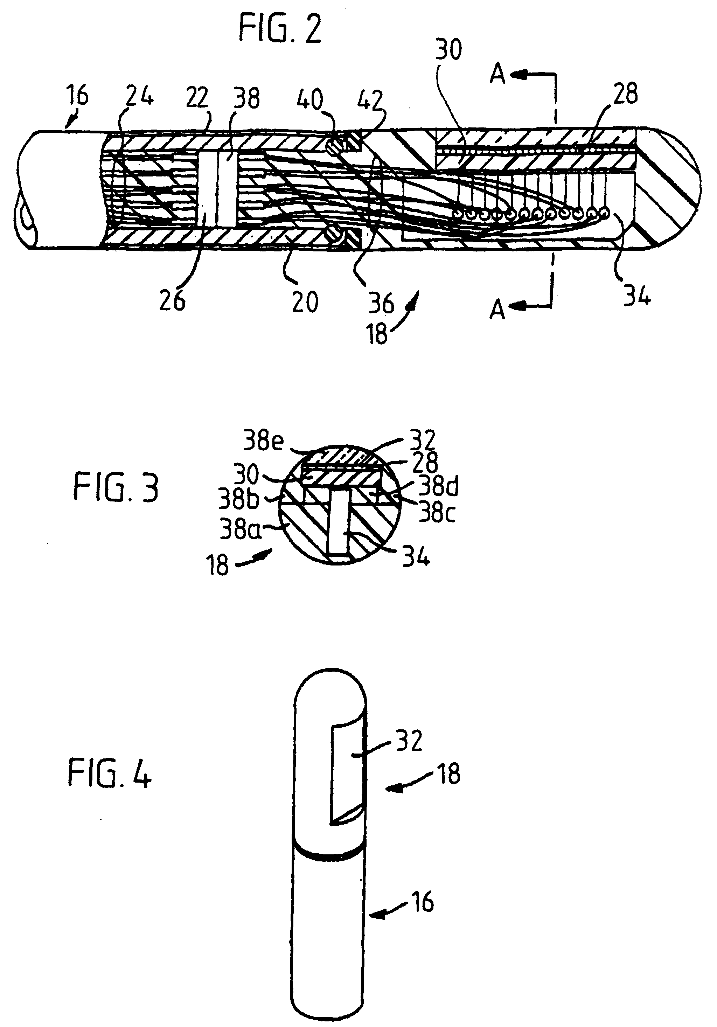

Wie in den Figuren 2 bis 4 detailliert dargestellt, wird die Ultraschall-Wandler-Sonde 18 an das distate Ende der Katheterhülle 16 angeschlossen. Die Katheterhülle 16 sollte vorzugsweise ein hohles Rohr 20 aus korrosionsfestem Material, wie z.B. rostfreiem Stahl mit einer Außenschicht 22 aus Werkstoff mit geringer Reibung, wie Teflon, enthalten. Eventuell könnte ein auf dem Markt verfügbares Kaufteil als Katheter verwendet werden. Mehrere Leitungen 24 laufen durch die Katheterhülle 16 vom distalen Ende bis zum Antrieb 12 auf der Konsole 10. Die Leitungen 24 laufen zu einem Stecker 26.

Die Sonde 18 enthält ein Array elektrisch getrennter Ultraschallelemente 28 aus Piezokeramik. Eine Grundplatte 30 wird rückwärtig am Array 28 montiert, so daß eine aktive Fläche 32 für die Emission der Ultraschallenergie entsteht. Eine gedruckte Schaltkarte 34 wird hinter die Grundplatte 30 montiert. Diese Karte 34 ist mit Schaltungen ausgestattet, die zu den einzelnen Elementen 28 geführt werden. Zu diesem Zweck könnte in der Grundplatte 30 eine Öffnung angebracht werden. Die Leitungen 36 verbinden die Anschlußflächen auf der gedruckten Schaltkarte 34 mit einer Steckerbuchse, in die der Stecker 22 eingesteckt wird.

Die Sonde 18 hat ein zylindrisches Gehäuse aus eingegossenen Teilen 38a, 38b, 38c, 38d, 38e mit einem abgerundeten distalen Ende und ist am Ende stufenförmig verjüngt zum Anschluß an die Katheterhülle. Die eingegossenen Teile sind untereinander verbunden und bilden so das Sondengehäuse, wie bereits erläutert. Die Gehäuseteile 38a, 38b, 38c, 38d sind aus biologisch nicht reaktivem Material, wie z.B. Urethan, das Gehäuseteil 38e besteht aus Ultraschallenergie durchlässigem Material, Teil 38e ist optionell; die Fläche 32 könnte als eine Außenfläche der Sonde 18 dienen. Der Stecker 38 wird am stufenförmig verjüngten Sonden-Gehäuseende angebracht, das in das hohle distale Ende der Katheterhülle eingepaßt ist. Ein Dichtringhalter 40 und eine Druckdichtung 42 sorgen für die Sicherung und die Dichtigkeit der Nahtstelle zwischen der Sonde 18 und der Katheterhülle 16. In einer typischen Ausführungsart beträgt die aktive Fläche des Arrays 3,8 mm x 1,5 mm bei einer Elementenanzahl von 24 plus 3 Schutzelemente an jedem Ende.

Die Katheterhülle 16 und die Sonde 18 müßten in den Körperhohlraum mit den bekannten Führungsdraht-Techniken eingeführt werden. Der Führungsdraht führt dann das distale Ende der Katheterhülle 16 und die Sonde 18 in den Körperhohlraum. Er könnte innen durch die Katheterhülle 16 und durch eine Öffnung (in der Zeichnung nicht sichtbar) im Gehäuse der Sonde 18 oder der Katheterhülle 16 geführt werden und die Sonde 18 könnte seitlich am Führungsdraht entlang verlaufen.

Die Katheterhülle 16 und die Sonde 18 haben in der Regel einen kleineren Durchmesser als der Körperhohlraum. Daher muß insbesondere dafür gesorgt werden, daß das distale Ende der Katheterhülle 16 und die Sonde 18 fest verankert sind, um Hin- und Herbewegungen oder axiale Bewegungen im Körperhohlraum während der Untersuchung der Gewebewand zu vermeiden. Zum Beispiel wäre es möglich, einen Ballon am distalen Ende der Katheterhülle 16 nach Einführung der Katheterhülle in den Körperhohlraum aufzupumpen. Dieser Ballon könnte an die Katheterhülle entweder mitdrehend befestigt oder mittels Kugellager befestigt werden, um sich bei Bewegungen der Katheterhülle nicht mitzudrehen. Eine andere Alternative wäre die Lösung, das distale Ende der Katheterhülle 16 durch eine weitere Hülle zu verankern, die den Zwischenraum zwischen dem distalen Ende der Katheterhülle 16 und der Gewebewand ausfüllt.

Am distalen Ende der Katheterhülle 16 schwingen Leitungen (24 und 36) mit der Hülle 16 und der Sonde 18. Am proximalen Ende der Katheterhülle 16 sind die Leitungen 24 jedoch mit dem stationären Gehäuse des Antriebs 12 verbunden. Daher bewegen sich die Leitungen 24 hin und her, wenn der Antrieb 12 in Bewegung ist. Die Katheterhülle 16 ist so lang, daß die Drehung pro Längeneinheit gering ist. In der Regel hat die Katheterhülle eine Länge von 50 - 100 cm und mehr. Um den elektrischen Anschluß zum Kabel 17 zu gewährleisten, könnten die Leitungen 24 durch die hohle Antriebswelle 14 und den Antrieb 12 geführt werden.

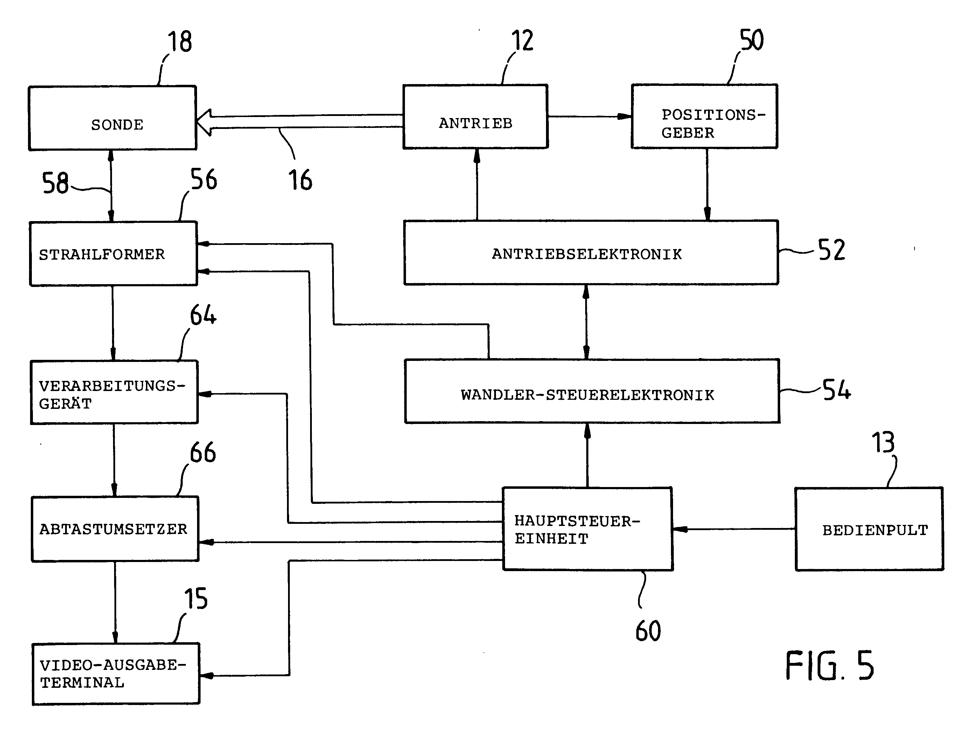

Fig. 5 ist ein Blockdiagramm des gesamten Ultraschall-Bildsystems mit Antrieb 12, Katheterhülle 16 und Sonde 18. Wie dargestellt, ist der Antrieb 12 durch die Katheterhülle 16 an die Sonde 18 gekoppelt, darüberhinaus ist er an einen Positionsgeber 50 angeschlossen. Die Antriebselektronik 52 steuert die Bewegungen des Antriebs 12 auf einen vom Positionsgeber 50 und der Wandler-Steuerelektronik 54 gelieferten Befehl hin. Die Sonde 18 ist elektrisch an einen Strahlformer 56 über Leitungen 36, Stecker (26 und 38), weitere Leitungen 24 und ein Slgnalkabel 17 angeschlossen, die insgesamt, wie in Fig. 5 dargestellt, durch den elektrischen Anschluß 58 dargestellt sind. Der Strahlformer 56 dient zur Fokussierung der übertragenen und/oder empfangenen Ultraschallenergie vorzugsweise durch Einführung von elektrischen Verzögerungen in den Signalwegen der Arrayelemente. Der Strahlformer 56 wird auf einen von der Wandler-Steuerelektronik 54 und der Hauptsteuereinheit 60 gelieferten Befehl hin aktiviert, wobei letztere durch ein Kommando vom Bedienpult 13 aktiviert wird. Die Signale zur Erzeugung des Sendeimpulses für die Sonde 18 werden in der Wandler-Steuerelektronik 54 erzeugt und sind daher über den Strahlformer 56 und die elektrische Anschlußeinheit 58 an die Sonde 18 gekoppelt. Die von der Sonde 18 erzeugten elektrischen Echosignale werden über den elektrischen Anschluß 58 und den Strahlformer 56 an ein Video-Verarbeitungsgerät 64 geliefert. Dieses Video-Verarbeitungsgerät 64 verarbeitet die hochfrequenten elektrischen Signale zu Bildsignalen, die an einen Abtastumsetzer 66 geliefert werden. Die Bildsignale werden abgespeichert und im Abtastumsetzer 66 zur Wiedergabe auf einem Video-Ausgabeterminal 15 entsprechend aufbereitet. Die Hauptsteuereinheit 60 steuert den Betrieb des Video-Verabreitungsgeräts 64, des Abtastumsetzers 66 und des Video-Ausgabeterminals 70 im üblichen Standardverfahren.

Es wird auf das US-Patent 5,140,558 Bezug genommen, dessen Beschreibung hier vollständig als Referenz zu Fig 5 angegeben wird. Der Positionsgeber 50, die Antriebssteuerungs-Elektronik 52, die Wandler-Steuerelektronik 54 der Strahlformer 56, das Video-Verarbeitungsgerät 64 und der Abtastumsetzer 68 sind in die Konsole 10 integriert (Fig. 1).

Wie im US-Patent 5,070,879 beschrieben, kann der Antrieb 12 so angesteuert werden, daß die Antriebswelle 14 sich kontinuierlich oder in bestimmten zeitlichen Abständen bewegt. Im allgemeinen liegt die Abbildungstiefe bei etwa 3 bis 4 cm und die Winkelgeschwindigkeit des Antriebs 12 bei 1 UPM. Auf jeden Fall wird der Antrieb mit dem Betrieb des Video-Ausgabeterminals 70 über die Hauptsteuereinheit 60 synchronisiert. Die Wandlerelemente 28 können sukzessiv angesteuert werden, um Abtastungen parallel zur Sondenachse 18 mit unterschiedlichen Winkeln um die Achse 18 durchzuführen, oder gleichzeitig zur Ausführung einer einzigen Abtastung senkrecht zur Sondenachse 18 angesteuert werden, wenn die Sonde 18 in Bewegung ist. Wenn die Wandlerelemente 28 bei einer kontinuierlichen Schwingungsbewegung der Sonde 18 sukzessiv aktiviert werden, können die dann ermittelten 3D-Daten auch dreidimensional auf dem Display dargestellt werden.

Das oben beschriebene Ausführungsbeispiel der Erfindung wird lediglich als bevorzugte Lösung zur besseren Veranschaulichung des Konzepts der Erfindung betrachtet; der Umfang der Erfindung beschränkt sich nicht nur auf dieses Ausführungsbeispiel. Zahlreiche weitere Varianten hierzu können von Fachleuten entwickelt werden, ohne daß man hierdurch den Rahmen dieser Erfindung verlassen würde. Zum Beispiel könnte die Katheterhülle 16 in ein nichtrotierendes Außenrohr integriert werden anstatt direkt in das Blutgefäß, wobei sich dann jedoch ein höherer Rohrdurchmesser ergeben würde.

## Patentansprüche

1. Endoluminales Ultraschallgerät mit einem Antrieb (12) mit drehhbarer, sich hin- und herbewegender Antriebswelle (14), einer flexiblen Katheterhülle (16), einem Ultraschallwandler (18) und durch die Katheterhülle (16) verlaufenden elektrischen Leitungen (24) zum elektrischen Anschluß des Ultraschallwandlers (18) an eine zu dessen Steuerung dienende Elektronik, **dadurch gekennzeichnet, daß** die Katheterhülle (16) an ihrem proximalen Ende mit der Antriebswelle (14) verbunden ist und mit ihr schwingt und der Ultraschallwandler (18) an das distale Ende der Katheterhülle (16) gekoppelt ist und mit dieser mitschwingt.

2. Gerät nach Anspruch 1, **dadurch gekennzeichnet, daß** der Ultraschallwandler (18) mit einem Array aus autonomen, über die Leitungen (24, 36) einzeln an die Elektronik anschließbaren, achsparallel angeordneten Wandlerelementen (28) ausgestattet ist.

3. Gerät nach Anspruch 2, **dadurch gekennzeichnet, daß** die Wandlerelemente (28) mittels der Elektronik sukzessiv ansteuerbar sind.

4. Gerät nach Anspruch 2, **dadurch gekennzeichnet, daß** die Wandlerelelemente (28) mittels der Elektronik gleichzeitig ansteuerbar sind.

5. Gerät nach einem der vorhergehenden Ansprüche, **dadurch gekennzeichnet, daß** die Elektronik mit Vorrichtungen zur Strahlfokussierung (56) zur Verbesserung der axialen Auflösung ausgestattet ist.

6. Gerät nach einem der vorhergehenden Ansprüche, **dadurch gekennzeichnet, daß** die Katheterhülle (16) eine Oberfläche aufweist, die mit einem reibungsarmen Material beschichtet ist.

## Claims

1. A endoluminal ultrasonic device with a drive (12) with rotatable forward- and backward-moving drive shaft (14), a flexible catheter sleeve (16), an ultrasonic transducer (18) and electrical leads (24) running through the catheter sleeve (16) for electrical connection of the ultrasonic transducer (18) to electronics serving to control it, **characterised in that** at its proximal end the catheter sleeve (18) is connected to the drive shaft (14) and vibrates with it and the ultrasonic transducer (18) is connected to the distal end of the catheter sleeve (16) and vibrates with it.

2. A device according to claim 1, **characterised in that** the ultrasonic transducer (18) is fitted with an array of autonomous transducer elements (28) which can be connected via the leads (24, 36) to the electronics and are arranged axially parallel.

3. A device according to claim 2, **characterised in that** the transducer elements (28) can be controlled successively by the electronics.

4. A device according to claim 2, **characterised in that** the transducer elements (28) can be controlled simultaneously by the electronics.

5. A device according to one of the preceding claims **characterised in that** the electronics is provided with beam focussing devices (56) to improve the axial resolution.

6. A device according to one of the preceding claims **characterised in that** the cathode sleeve (16) has a surface which is coated with a low-friction material.

## Revendications

1. Appareil endoluminal à ultrasons avec un entraînement (12) avec un arbre d'entraînement rotatoire (14) à va-et-vient, une gaine de cathéter flexible (16), un convertisseur d'ultrasons (18) et des câbles électriques (24) traversant la gaine de cathéter (16) pour le raccordement électrique du convertisseur d'ultrasons (18) à une électronique servant à sa commande, **caractérisé en ce que** la gaine de cathéter (16) est reliée, à son extrémité proximale, à l'arbre d'entraînement (14) et vibre en même temps que celui-ci et que le convertisseur d'ultrasons (18), à l'extrémité distale, est couplé à la gaine de cathéter (16) et vibre avec celle-ci.

2. Appareil selon la revendication 1, **caractérisé en ce que** le convertisseur d'ultrasons (18) est constitué par une série d'éléments convertisseurs (28) pouvant être connectés individuellement par les câbles (24, 36) à l'électronique et disposés parallèlement à l'axe.

3. Appareil selon la revendication 2, **caractérisé en ce que** les éléments convertisseurs (28) peuvent être commandés successivement au moyen de l'électronique.

4. Appareil selon la revendication 2, **caractérisé en ce que** les éléments convertisseurs (28 peuvent être commandés simultanément au moyen de l'électronique.

5. Appareil selon une des revendications précédentes, **caractérisé en ce que** l'électronique est équipée de dispositifs pour la focalisation des rayons (56) pour l'amélioration de la résolution axiale.

6. Appareil selon une des revendications précédentes, **caractérisé en ce que** la gaine de cathéter (16) présente une surface qui est revêtue d'une matière à faible friction.
